# EUROPÄISCHE PATENTSCHRIFT

(11) **EP 2 978 391 B1**
(45) Veröffentlichungstag und Bekanntmachung des Hinweises auf die Patenterteilung: **31.01.2018**
(21) Anmeldenummer: 14714594.0
(22) Anmeldetag: 27.03.2014
(51) Int. Cl.: A61F 5/01

(54) **BEFESTIGUNGSSYSTEM**
ATTACHMENT SYSTEM
SYSTEME DE FIXATION

(30) Priorität: 28.03.2013 DE 102013005366
(43) Veröffentlichungstag der Anmeldung: 03.02.2016
(73) Patentinhaber: Otto Bock HealthCare GmbH, 37115 Duderstadt (DE)
(72) Erfinder: SCHILLING, Matthias, 37345 Weißenborn-Lüderode (DE); HOCHMANN, David, 12247 Berlin (DE)
(74) Vertreter: Friedrich, Andreas
(86) Internationale Anmeldenummer: PCT/EP2014/000828
(87) Internationale Veröffentlichungsnummer: WO 2014/154359

(56) Entgegenhaltungen:
- DE-U1-202013 000 384
- US-A- 3 732 861
- US-A- 4 237 873
- US-A- 4 428 094
- US-A1- 2012 283 613

## Beschreibung

Die Erfindung betrifft ein Befestigungssystem mit einem ersten Bauteil und einem zweiten Bauteil, die Bestandteile einer orthopädietechnischen Einrichtung sind, zum Befestigen des ersten Bauteiles an dem zweiten Bauteil, wobei das erste Bauteil eine erste Anlagefläche und eine erste Erstreckungsrichtung und das zweite Bauteil eine zweite Anlagefläche und eine zweite Erstreckungsrichtung aufweist und ein Einschlusswinkel zwischen der ersten Erstreckungsrichtung und der zweiten Erstreckungsrichtung innerhalb eines Winkelbereichs stufenlos einstellbar ist, wobei der Winkelbereich in einer Ebene liegt, die durch die erste Anlagefläche definiert wird und das Befestigungssystem wenigstens ein Kraftübertragungselement aufweist, das im befestigten Zustand der beiden Bauteilen zwischen den beiden Anlageflächen eingeklemmt ist.

Ein ähnliches Befestigungssystem ist aus der US 3,732,861 A bekannt.

Derartige Befestigungssysteme sind aus dem Stand der Technik seit langem bekannt. Sie werden beispielsweise in orthopädietechnischen Einrichtungen, wie Orthesen und Prothesen verwendet, um die jeweilige Einrichtung an die individuellen körperlichen Gegebenheiten des späteren Trägers der Orthese oder Prothese anzupassen. So wird beispielsweise bei Bein- und Knöchelorthesen oftmals ein Schienensystem verwendet, das über Gelenke verfügt, zwischen denen sich die einzelnen Schienen befinden. Die individuelle Anpassung erfolgt dabei nicht nur über eine Längenänderung der zwischen den einzelnen Gelenken vorhandenen Schienen, sondern auch beispielsweise durch eine Veränderung der Stellung beispielsweise des Knöchelgelenkes in einer belastungsfreien Nullstellung. Daher ist es wichtig, die beiden Bauteile in einem individuell einstellbaren Winkel aneinander anordnen zu können. Insbesondere bei der Verwendung als Knöchelorthese, jedoch auch bei anderen Verwendungsmöglichkeiten, ist diese Verbindung zwischen den beiden Bauteilen erheblichen mechanischen Belastungen insbesondere durch große auftretende Drehmomente ausgesetzt. Sind die beiden Bauteile einmal miteinander verbunden, sollen sie sich jedoch bei der normalen Benutzung der jeweiligen Einrichtung nicht gegeneinander verschieben und insbesondere nicht den Einschlusswinkel verändern. Daher muss die Verbindung stabil ausgebildet sein.

Aus dem Stand der Technik ist bekannt, die beiden miteinander zu verbindenden Bauteile so auszuformen, dass sie entlang einer Seitenfläche aneinander anliegen. Wird nun beispielsweise eine Orthese an die individuellen Bedürfnisse ihres Trägers angepasst, wird der Winkel, in dem diese Seitenfläche relativ zu einem der Bauteile verläuft, durch einen speziellen Zuschnitt des jeweiligen Bauteils verändert, so dass die Außenkontur des jeweiligen Bauteils nun als Anschlag für diesen Einschlusswinkel dient. Dies hat mehrere Nachteile. Zum einen sind bauliche Veränderungen an dem einen Bauteil nötig, um eine optimale Anpassung zu erreichen. Zum anderen ist es nur in sehr begrenztem Umfang möglich, eine fehlerhafte Bearbeitung auszugleichen, so dass für diesen Fall das Bauteil entsorgt und durch ein anderes frisches Bauteil ersetzt werden muss, das erneut der zusätzlichen Bearbeitung unterworfen werden muss. Insbesondere für den Fall, dass sich die einmal gewählte Passform und der einmal gewählte Einschlusswinkel nicht als die optimale Wahl erweisen, ist es oftmals nicht möglich, einfache Änderungen dieser Einstellungen vorzunehmen.

Die gewählte Anschlagskontur ist bei den Befestigungssystemen aus dem Stand der Technik jedoch notwendig, um den auftretenden mechanischen Belastungen standhalten zu können.

Alternativ dazu ist auch eine stufenweise Verstellung des gewählten Einschlusswinkels bekannt, wobei die beiden aneinander zu befestigenden Bauteile beispielsweise verschraubt, vernietet oder verstiftet werden können. Hier ist zwar eine nachträgliche Änderung insbesondere bei einer Verschraubung der beiden Bauteile möglich, ohne dass jedoch eine stufenlose Einstellbarkeit des Einschlusswinkels gegeben wäre. Um für jeden Träger der Orthese oder Prothese die optimale Einstellung zu erreichen, ist die stufenlose Einstellbarkeit jedoch oftmals notwendig.

Aus der DE 20 2013 000 384 U1 ist ein Befestigungssystem zum Befestigen eines ersten Bauteils an einem zweiten Bauteil bekannt, bei dem ein Sensor, insbesondere eine Kamera, befestigt und justiert werden soll. Die US 4,428,094 A beschreibt ein Befestigungssystem für das Gelenkelement eines Brillenbügels.

Die US 4 237 873 offenbart ein System, bei dem zwischen den beiden Bauteilen ein vollflächiges Verbindungselement in Form einer Zwischenlegscheibe angeordnet ist. Die US 2012/0283613 A1 hingegen offenbart ein System, bei dem ein elastisch verformbarer Ball beim Befestigen der beiden Bauteile aneinander verformt wird und so ein ihn umgebendes Reibungsband in Kontakt mit einer Innenfläche einer Aufnahme drückt, in der der Ball angeordnet ist.

Der Erfindung liegt daher die Aufgabe zugrunde, ein Befestigungssystem so zu verbessern, dass es trotz einem kleinen Bauraum eine stufenlose Einstellung des Einschlusswinkels erlaubt, die zu erwartenden auftretenden mechanischen Kräfte aufnehmen kann und dennoch eine einfach lösbare Befestigung der beiden Bauteile aneinander bietet.

Die Erfindung löst die gestellte Aufgabe durch ein Befestigungssystem gemäß dem Oberbegriff des Anspruchs 1, das sich dadurch auszeichnet, dass das Befestigungssystem ein Klemmelement aufweist, durch das das erste Bauteil an dem zweiten Bauteil befestigbar ist, wenn die erste Anlagefläche an der zweiten Anlagefläche anliegt. Es ist auch möglich, genau ein Kraftübertragungselement zu verwenden. Durch diese Ausgestaltung des Befestigungssystems wird einerseits durch das Klemmelement eine leicht lösbare Verbindung zwischen den beiden Bauteilen geschaffen. Andererseits wird zudem durch das Kraftübertragungselement gewährleistet, dass die ansonsten zu schwach ausfallende Klemmbefestigung den mechanischen Belastungen, die auftreten, standhalten kann. Dies ist bei einer herkömmlichen Schraubklemmung nicht der Fall, so dass die Verwendung eines Klemmelementes allein nicht geeignet wäre, das entsprechende Befestigungssystem für die mechanischen Belastungen auszulegen.

Durch das erfindungsgemäße Befestigungssystem werden das erste Bauteil und das zweite Bauteil so aneinander befestigt und festgelegt, dass unter den zu erwartenden auftretenden Kräften eine Bewegung der beiden Bauteile relativ zueinander nicht mehr möglich ist. Diese zu erwartenden auftretenden Kräfte sind natürlich von der Verwendung, für die die Bauteile vorgesehen sind, abhängig. Werden beispielsweise zwei Bauteile einer Knöchelorthese miteinander verbunden, treten bei bestimmungsgemäßem Gebrauch deutlich größere Kräfte auf, als dies beispielsweise bei einer Armorthese der Fall ist. Entsprechend müssen die jeweiligen Anlageflächen und/oder das Kraftübertragungselement ausgebildet sein, um den zu erwartenden mechanischen Belastungen standhalten zu können.

Vorzugsweise weist das Kraftübertragungselement eine geschlossene Kontur auf. Besonders vorteilhafterweise ist das Kraftübertragungselement ein O-Ring.

Soll nun das erste Bauteil an dem zweiten Bauteil befestigt werden, wenn beispielsweise eine Orthese oder Prothese an den Körper des Trägers individuell angepasst werden soll, werden die erste Anlagefläche und die zweite Anlagefläche aneinander angelegt. In diesem Zustand ist eine Verschiebung und insbesondere Verschwenkung der beiden Bauteile gegeneinander möglich, so dass der Einschlusswinkel zwischen den beiden Erstreckungsrichtungen der beiden Bauteile stufenlos eingestellt werden kann. Ist ein gewünschter Einschlusswinkel erreicht, wird das Klemmelement angezogen und die beiden Bauteile miteinander verbunden. Für den Fall, dass ein derartiges Befestigungssystem ohne Kraftübertragungselement verwendet wird, entscheidet die durch das Klemmelement aufgebrachte Klemmkraft und damit die Haftreibung zwischen den beiden Anlageflächen, ob das Befestigungssystem einer aufgebrachten Kraft oder einem Drehmoment standhält oder ob es zu einer Verschiebung oder Verschwenkung der beiden Bauteile relativ zueinander kommt. Der tatsächliche Kraftfluss zwischen den beiden aneinander anliegenden Kontaktflächen erfolgt dabei jedoch nicht homogen über die gesamte Fläche der Anlagefläche, sondern ist aufgrund von Oberflächenrauhigkeiten in einigen Bereichen verstärkt und in anderen Bereichen abgeschwächt. Position und Ausmaß dieser Bereiche ist fertigungstechnisch nicht zu kontrollieren, so dass eine Abschätzung der maximalen Haftkraft nicht möglich ist.

Erst durch die Verwendung eines Kraftübertragungselementes zwischen den beiden Anlageflächen ist ein reproduzierbarer und voreinstellbarer Verlauf des Kraftflusses möglich. Dabei hat sich herausgestellt, dass durch die geschlossene Kontur des Kraftübertragungselementes die Haftkraft zwischen dem Kraftübertragungselement und den beiden Anlageflächen maximiert werden kann. Durch geschickte Wahl der geometrischen Form und/oder des Materials, aus dem das Kraftübertragungselement besteht, kann die Haftkraft und damit die mechanische Stabilität der Befestigung der beiden Bauteile aneinander beeinflusst und optimiert werden.

Die Erstreckungsrichtung des jeweiligen Bauteils kann beispielsweise die Richtung einer Seitenfläche oder die Richtung der größten Ausdehnung des jeweiligen Bauteils sein. Dies ist für die Funktionsweise des Befestigungssystems unerheblich, da es lediglich darauf ankommt, das für einmal gewählte Erstreckungsrichtungen der zwischen diesen beiden Richtungen eingeschlossene Einschlusswinkel in einem Winkelbereich stufenlos einstellbar ist. Dabei wird die Größe des Winkelbereiches nicht durch die Wahl der Erstreckungsrichtungen beeinflusst, während die absoluten Werte des jeweiligen Einschlusswinkels natürlich von dieser Wahl abhängen können.

Vorteilhafterweise liegt der Winkelbereich in einer Ebene, die von der ersten Anlagefläche definiert wird. Eine Veränderung des Einschlusswinkels bedeutet also eine Verschwenkung der beiden Bauteile relativ zueinander um eine Schwenkachse, die senkrecht auf der ersten Anlagefläche liegt.

Vorzugsweise verläuft das Klemmelement durch eine Bohrung in der ersten Anlagefläche und in der zweiten Anlagefläche. Dies hat zum einen zur Folge, dass das Befestigungssystem besonders klein ausgebildet sein kann, und führt zum anderen dazu, dass die beiden Bohrungen in der ersten Anlagefläche und der zweiten Anlagefläche zur Befestigung der beiden Bauteile aneinander in Überdeckung gebracht werden müssen, was eine besonders einfache Ausrichtung der beiden Bauteile zueinander ermöglicht.

Vorteilhafterweise weist das Klemmelement daher eine Gewindeplatte auf, die an einer der jeweiligen Anlagefläche entgegen gesetzten Seite des Bauteils positionierbar ist. In diese Gewindeplatte greift beispielsweise eine Schraube ein, die durch die beiden Bohrungen in der ersten Anlagefläche und der zweiten Anlagefläche und damit durch die beiden Bauteile hindurch verläuft und beispielsweise mit einem Schraubenkopf oder einer Unterlegscheibe an der Austrittsseite des jeweiligen Bauelementes anliegt. Wird nun die Schraube angezogen, wird eine Klemmkraft aufgebracht, so dass das Kraftübertragungselement zwischen den beiden Bauteilen bzw. deren Anlageflächen eingeklemmt wird und es so zu einem wohl definierten und reproduzierbar ausgestalteten Kraftfluss kommt. Zum Verstellen des Einflusswinkels zwischen den beiden Erstreckungsrichtungen der beiden Bauteile ist es in diesem Fall ausreichend, die Schraube etwas zu lösen und somit die Klemmkraft zu verringern, so dass eine Verschwenkung der beiden Bauteile relativ zueinander möglich wird. Die Schwenkachse verläuft dabei vorteilhafterweise zentral durch das Klemmelement, das durch die Bohrungen in den beiden Bauteilen und den beiden Anlageflächen verläuft. Das Klemmelement besteht in diesem Fall aus der Gewindeplatte und der Schraube. Alternative Klemmelemente umfassen eine Schraube und eine Schraubenmutter und gegebenenfalls eine Unterlegscheibe. Natürlich sind auch andere Möglichkeiten einer Klemmverbindung denkbar, die für andere Anwendungsbereiche von Vorteil sein können.

Vorzugsweise weist das Klemmelement, und insbesondere die Gewindeplatte, wenigstens einen Anschlag auf, der im befestigten Zustand an wenigstens einem Gegenanschlag an einem der Bauteile anliegt. Dadurch wird zusätzlich zu der aufgebrachten Klemmkraft und der zwischen den Anlageflächen und der Kraftübertragungselement wirkenden Haftkraft erreicht, dass eine Verschwenkung der beiden Bauteile relativ zueinander im aneinander befestigten Zustand erschwert wird. Damit wird folglich ein Befestigungssystem erreicht, dass noch größeren mechanischen Belastungen standhält und somit in einen großen Einsatzbereich einsetzbar ist.

Vorzugsweise verläuft das Klemmelement im befestigten Zustand der beiden Bauteile durch das Kraftübertragungselement hindurch. Das Kraftübertragungselement weist eine geschlossene Kontur auf und ist somit ringförmig ausgebildet. Als besonders vorteilhaft hat sich die Verwendung eines O-Ringes erwiesen. Durch diesen Ring verläuft auch das Klemmelement.

Vorteilhafterweise besteht das Kraftübertragungselement aus einem elastischen Material, beispielsweise Gummi. Dies hat zur Folge, dass das Kraftübertragungselement beim Einklemmen zwischen den beiden Anlageflächen verformt wird und so die Haftreibung zwischen den beiden Anlageflächen und dem Kraftübertragungselement maximiert wird. Zudem ist es dem Kraftübertragungselement in dieser Ausgestaltung möglich, beispielsweise vorhandenen Unebenheiten und Oberflächenrauhigkeiten zu folgen und so über seinen gesamten Umfang zur Übertragung der Haltekraft beizutragen.

In einer besonders bevorzugten Ausgestaltung der vorliegenden Erfindung ist in der ersten Anlagefläche und/oder der zweiten Anlagefläche eine Nut angeordnet, in die das Kraftübertragungselement eingelegt werden kann. Damit wird ein Verrutschen oder Verschieben des Kraftübertragungselementes während des aneinander Befestigen der beiden Bauteile verhindert. Zudem kann das Kraftübertragungs-element beispielsweise in der Nut befestigt werden, in dem es beispielsweise angeklebt wird. Damit ist sichergestellt, dass das Kraftübertragungselement nicht beispielsweise beim Transport verlorengeht. Ein Benutzer des Befestigungssystems bekommt folglich immer ein funktionsfähiges System, ohne dass das Risiko besteht, dass das für die Funktionsweise des Systems notwendige Kraftübertragungselement verlorengeht. Diese Nut verläuft vorzugsweise möglichst weit von der Bohrung, durch die das Klemmelement verläuft, entfernt. Dadurch wird einerseits erreicht, dass angreifende Drehmomente möglichst weit von der potentiellen Schwenkachse entfernt angreifen und andererseits ein möglichst großes Kraftübertragungselement verwendet werden kann. Aufgrund der erhöhten Haftreibung zwischen dem Kraftübertragungselement und der jeweiligen Anlagefläche ist ein möglichst großer Kontaktbereich zwischen diesen Bauteilen für die Stabilität der Befestigung vorteilhaft.

Wie bereits dargelegt ist das erste Bauteil und das zweite Bauteil erfindungsgemäß ein Bestandteil einer orthopädietechnischen Einrichtung, insbesondere eine Prothese oder Orthese. Vorzugsweise ist die orthopädietechnische Einrichtung ein Gelenk.

Bei der vorliegenden Erfindung muss das Befestigungssystem nicht als separates Bauteil ausgebildet sein, sondern beinhaltet spezielle Ausgestaltungen des ersten Bauteils und des zweiten Bauteils. Diese umfassen beispielsweise die beiden Anlageflächen, die so ausgebildet sein sollten, dass sie vollflächig aneinander anliegen können. Beispielsweise bei einer Knöchelorthese wird ein Knöchelgelenk verwendet, an dessen distalen Ende eine Fußbaugruppe angeordnet wird, die zur Stützung des Fußes
dient. Dabei muss der Winkel, in dem diese beiden Bauteile, also die Fußbaugruppe und das Gelenk, aneinander angeordnet werden, auf die jeweiligen individuellen Eigenschaften des Trägers abgestimmt werden. Um ein erfindungsgemäßes Befestigungssystem in diesem Fall verwenden zu können, sollte das erste Bauteil, das in diesem Fall beispielsweise durch das Gelenk gebildet wird, und das zweite Bauteil, das durch die Fußbaugruppe gebildet wird, die beiden Anlageflächen aufweisen. Ein zusätzliches Zwischenbauteil, das einerseits mit dem ersten und andererseits mit dem zweiten Bauteil auf eine andere Weise verbunden wird, wird so sicher vermieden, so dass eine besonders einfache, kostengünstige, schnelle und verlässliche Befestigung möglich ist.

Sofern eines der beiden Bauteile ein Bestandteil eines Gelenks, beispielsweise einer der schwenkbaren Schenkel eines Gelenks, ist, wird durch die Befestigung der beiden Bauteile aneinander die Schwenkbarkeit des Gelenks nicht beeinträchtigt. Weist das Gelenk beispielsweise zwei relativ zueinander verschwenkbare Schenkel auf, wird das zweite Bauteil beispielsweise nur durch einen dieser beiden Schenkel gebildet. An diesem wird ein erstes Bauteil, das beispielsweise eine Schiene oder ein ähnliches Element sein kann, angeordnet. Damit wird eine starre Befestigung der Schiene an dem Schenkel des Gelenks erreicht, ohne dass dadurch die Verschwenkbarkeit der beiden Schenkel des Gelenks relativ zueinander beeinträchtigt würde. Ein Befestigungssystem gemäß einem Ausführungsbeispiel der vorliegenden Erfindung ist überall dort von Vorteil, wo zwei Bauteile in einem bestimmten Winkel und in einer bestimmten Position relativ zueinander befestigt werden sollen, so dass sie in diesem Zustand nicht mehr unter den zu erwartenden Kräften gegeneinander bewegt werden können. Der tatsächlich gewählte Winkel beziehungsweise die tatsächlich vorhandene Position kann dabei individuell eingestellt werden. Dies ist nicht nur bei Orthesen und Prothesen, sondern beispielsweise auch bei der individuellen Anpassung von Rollstühlen oder ähnlichen Hilfsmitttein vorteilhaft.

Mit Hilfe einer Zeichnung wird nachfolgend ein Ausführungsbeispiel der vorliegenden Erfindung näher erläutert. Es zeigt
- Figur 1: - ein Befestigungssystem gemäß einem ersten Ausführungsbeispiel der vorliegenden Erfindung in einer Explosionsdarstellung,
- Figur 2 bis 4: - das Befestigungssystem aus Figur 1 im montierten Zustand in unterschiedlichen Ansichten.

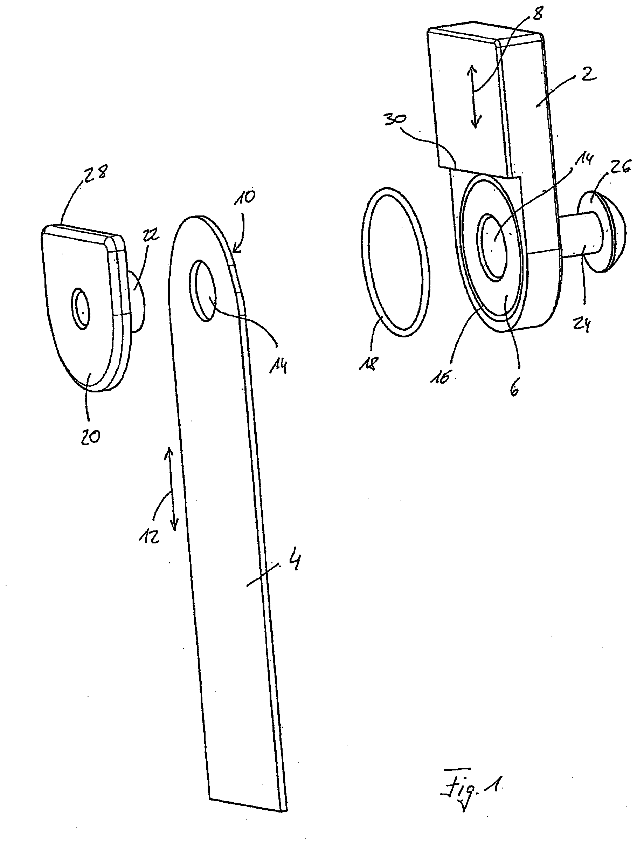

Figur 1 zeigt ein Befestigungssystem mit einem ersten Bauteil 2 und einem zweiten Bauteil 4. Das erste Bauteil 2 weist eine erste Anlagefläche 6 sowie eine erste Erstreckungsrichtung 8 auf. Das zweite Bauteil 4 verfügt über eine zweite Anlagefläche 10 sowie eine zweite Erstreckungsrichtung 12. Sowohl das erste Bauteil 2 als auch das zweite Bauteil 4 verfügen jeweils über eine Bohrung 14, die auch durch die jeweilige Anlagefläche 6, 10 verläuft. Um das erste Bauteil 2 und das zweite Bauteil 4 aneinander zu befestigen, werden die beiden Bohrungen 14 in Überdeckung gebracht.

In der ersten Anlagefläche 6 befindet sich eine Nut 16, in die ein Kraftübertragungselement 18, das im gezeigten Ausführungsbeispiel als O-Ring ausgeführt ist, eingelegt werden kann.

Das Befestigungssystem verfügt über ein Klemmelement, das aus einer Gewindeplatte 20, die über einen Vorsprung 22 verfügt, in dem sich das Gewinde befindet, sowie über eine Schraube 24 verfügt. Zum Befestigen der beiden Bauteile 2, 4 aneinander wird die Schraube 24 durch die beiden Bohrungen 14 hindurch in den Vorsprung 22 eingeschraubt. Ein Schraubenkopf 26 kommt dabei am ersten Bauteil 2 zur Anlage, während die Gewindeplatte 20 am zweiten Bauteil 4 anliegt. Durch die Schraube 24 und die Gewindeplatte 20 wird eine Klemmkraft auf die beiden Bauteile 2, 4 und insbesondere auf das Kraftübertragungselement 18 ausgeübt.

Die in Figur 1 gezeigte Gewindeplatte 20 verfügt über einen Anschlag 28, der im verbundenen Zustand mit einem am ersten Bauteil 2 angeordneten Gegenanschlag 30 zusammenwirkt.

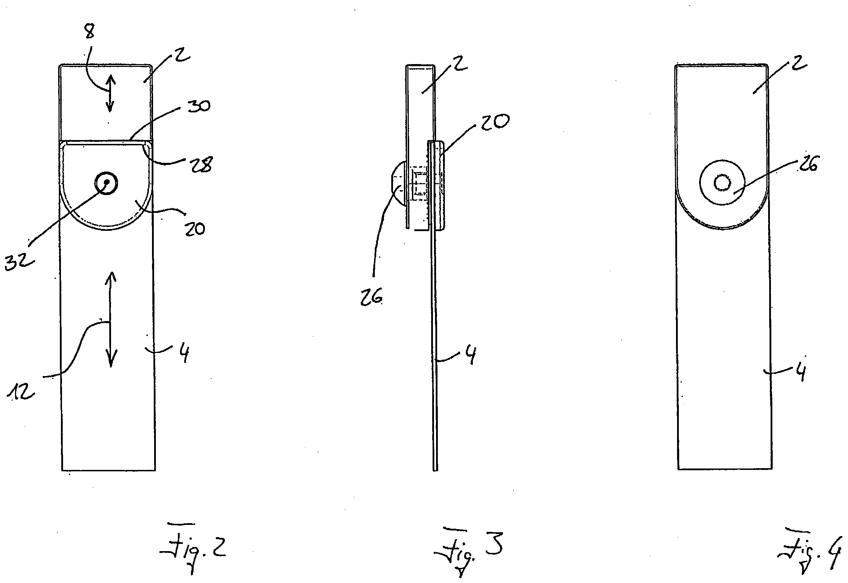

In den Figuren 2 bis 4 ist das in Figur 1 gezeigte Befestigungssystem im zusammengesetzten Zustand aus unterschiedlichen Ansichten erkennbar. Figur 2 zeigt das erste Bauteil 2 und das zweite Bauteil 4, wobei auch die Gewindeplatte 20 dargestellt ist, die mit dem Anschlag 28 am Gegenanschlag 30 anliegt. Man erkennt, dass die beiden Erstreckungsrichtungen 8, 12 parallel zueinander verlaufen, so dass der Einschlusswinkel in diesem Fall 180 Grad beträgt. Durch ein einfaches Lösen des Klemmelementes, also insbesondere der Schraube 24 in den Vorsprung 22 der Gewindeplatte 20 wird die Klemmkraft verringert, so dass das erste Bauteil 2 und das zweite Bauteil 4 relativ zueinander verschwenkt werden können. Dabei verläuft eine Schwenkachse 32 in Figur 2 aus der Zeichenebene heraus zentral durch die beiden Bohrungen 14 und das aus Schraube 24 und Gewindeplatte 22 bestehende Klemmelement. Dabei verschwenkt das zweite Bauteil 4 nicht nur relativ zum ersten Bauteil 2, sondern auch relativ zur Gewindeplatte 20, so dass unabhängig vom eingeschlossenen Einschlusswinkel der Anschlag 28 am Gegenanschlag 30 anliegt, und so im befestigten Zustand eine weitere Verschwenkung mit verhindert.

Figur 3 zeigt das Befestigungssystem in einer Seitenansicht. Das erste Bauteil 2 und das zweite Bauteil 4 werden durch die Gewindeplatte 20 und die Schraube 24 mit dem Schraubenkopf 26 eingeklemmt.

Figur 4 zeigt das Befestigungssystem in einer Rückansicht, so dass nur das erste Bauteil 2, das zweite Bauteil 4 und der Schraubenkopf 26 zu erkennen sind.

### Bezugszeichenliste

### Bezugszeichenliste

- 2: Erstes Bauteil
- 4: Zweites Bauteil
- 6: Erste Anlagefläche
- 8: Erste Erstreckungsrichtung
- 10: Zweite Anlagefläche
- 12: Zweite Erstreckungsrichtung
- 14: Bohrung
- 16: Nut
- 18: Kraftübertragungselement
- 20: Gewindeplatte
- 22: Vorsprung
- 24: Schraube
- 26: Schraubenkopf
- 28: Anschlag
- 30: Gegenanschlag
- 32: Schwenkachse

## Patentansprüche

1. Befestigungssystem mit einem ersten Bauteil (2) und einem zweiten Bauteil (4), die Bestandteile einer Orthese oder Prothese sind, zum Befestigen des ersten Bauteils (2) an dem zweiten Bauteil (4), wobei
das erste Bauteil (2) eine erste Anlagefläche (6) und eine erste Erstreckungsrichtung (8) und
das zweite Bauteil (4) eine zweite Anlagefläche (10) und eine zweite Erstreckungsrichtung (12) aufweist **dadurch gekennzeichnet, dass** ein Einschlusswinkel zwischen der ersten Erstreckungsrichtung (8) und der zweiten Erstreckungsrichtung (12) innerhalb eines Winkelbereichs, der in einer Ebene liegt, die durch die erste Anlagefläche (6) definiert wird stufenlos einstellbar ist, wobei das Befestigungssystem wenigstens ein Kraftübertragungselement (18) aufweist, das im befestigten Zustand der beiden Bauteile (2, 4) zwischen den beiden Anlageflächen (6, 10) eingeklemmt ist,
wobei das Befestigungssystem ein Klemmelement aufweist, durch das das erste Bauteil (2) an dem zweiten Bauteil (4) befestigbar ist, wenn die erste Anlagefläche (6) an der zweiten Anlagefläche (10) anliegt.

2. Befestigungssystem nach Anspruch 1, **dadurch gekennzeichnet, dass** das Kraftübertragungselement (18) eine geschlossene Kontur aufweist, insbesondere ein O-Ring ist.

3. Befestigungssystem nach Anspruch 1 oder 2, **dadurch gekennzeichnet, dass** das Klemmelement durch eine Bohrung (14) in der ersten Anlagefläche (6) und der zweiten Anlagefläche (10) verläuft.

4. Befestigungssystem nach einem der vorstehenden Ansprüche, **dadurch gekennzeichnet, dass** das Klemmelement eine Gewindeplatte (20) aufweist, die an einer der jeweiligen Anlagefläche (6, 10) entgegen gesetzten Seite eines Bauteils (2, 4) positionierbar ist.

5. Befestigungssystem nach Anspruch 4, **dadurch gekennzeichnet, dass** das Klemmelement, insbesondere die Gewindeplatte (20), wenigstens einen Anschlag (28) aufweist, der im befestigten Zustand an wenigstens einem Gegenanschlag (30) an einem der Bauteile (2, 4) anliegt.

6. Befestigungssystem nach einem der Ansprüche 3 bis 5, **dadurch gekennzeichnet, dass** das Klemmelement im befestigten Zustand der beiden Bauteile (2, 4) durch das Kraftübertragungselement (18) hindurch verläuft.

7. Befestigungssystem nach einem der vorstehenden Ansprüche, **dadurch gekennzeichnet, dass** das Kraftübertragungselement (18) aus einem elastischen Material, beispielsweise Gummi, besteht.

8. Befestigungssystem nach einem der vorstehenden Ansprüche, **dadurch gekennzeichnet, dass** in der ersten Anlagefläche (6) und/oder der zweiten Anlagefläche (10) eine Nut (16) zum Aufnehmen des Kraftübertragungselementes 18 angeordnet ist.

9. Befestigungssystem nach einem der vorstehenden Ansprüche, **dadurch gekennzeichnet, dass** die orthopädietechnische Einrichtung ein Gelenk ist.

## Claims

1. A fastening system including a first component (2) and a second component (4), which are parts of an orthosis or prothesis, for fastening the first component (2) to the second component (4), wherein
the first component (2) has a first bearing surface (6) and a first direction of extent (8), and
the second component (4) has a second bearing surface (10) and a second direction of extent (12), **characterized in that**
an included angle between the first direction of extent (8) and the second direction of extent (12) is adjustable steplessly within an angle range, which lies in a plane which is defined by the first bearing surface (6), wherein
the fastening system has at least one force-transmitting element (18) which is in the fastened state of the two components (2, 4) clamped between the two bearing surfaces (6, 10), wherein
the fastening system has a clamp element with which the first component (2) can be fastened to the second component (4) when the first bearing surface (6) lies on the second bearing surface (10).

2. The fastening system as claimed in claim 1, **characterized in that** the force-transmitting element (18) has a closed contour and in particular is an O-ring.

3. The fastening system as claimed in claim 1 or 2, **characterized in that** the clamp element extends through a bore (14) in the first bearing surface (6) and the second bearing surface (10).

4. The fastening system as claimed in one of the preceding claims, **characterized in that** the clamp element has a threaded plate (20), which can be positioned on a side of a component (2, 4) opposite the respective bearing surface (6, 10).

5. The fastening system as claimed in claim 4, **characterized in that** the clamp element, in particular the threaded plate (20), has at least one stop (28) which in the fastened state lies on at least one counter-stop (30) on one of the components (2, 4).

6. The fastening system as claimed in one of claims 3 to 5, **characterized in that** the clamp element extends through the force-transmitting element (18) in the fastened state of the two components (2, 4).

7. The fastening system as claimed in one of the preceding claims, **characterized in that** the force-transmitting element (18) is made of an elastic material, for example rubber.

8. The fastening system as claimed in one of the preceding claims, **characterized in that** a groove (16) for receiving the force-transmitting element (18) is arranged in the first bearing surface (6) and/or the second bearing surface (10).

9. The fastening system as claimed in one of the preceding claims, **characterized in that** the orthopedic device is a joint.

## Revendications

1. Système de fixation comprenant un premier composant (2) et un deuxième composant (4) qui font partie intégrante d'une orthèse ou d'une prothèse, destiné à fixer le premier composant (2) au deuxième composant (4), dans lequel
le premier composant (2) présente une première surface de contact (6) et une première direction d'allongement (8) et
le deuxième composant (4) présente une deuxième surface de contact (10) et une deuxième direction d'allongement (12),
**caractérisé en ce que**
un angle inclus entre la première direction d'allongement (8) et la deuxième direction d'allongement (12) est réglable en continu à l'intérieur d'une plage angulaire se trouvant sur un plan défini par la première surface de contact (6),
dans lequel le système de fixation présente au moins un élément de transmission des forces (18) qui est serré entre les deux surfaces de contact (6, 10) lorsque les deux composants (2, 4) se trouvent à l'état fixé,
dans lequel le système de fixation présente un élément de serrage au moyen duquel le premier composant (2) peut être fixé au deuxième composant (4) lorsque la première surface de contact (6) est en contact avec la deuxième surface de contact (10).

2. Système de fixation selon la revendication 1, **caractérisé en ce que** l'élément de transmission des forces (18) présente un contour fermé, et est en particulier un joint torique.

3. Système de fixation selon la revendication 1 ou 2, **caractérisé en ce que** l'élément de serrage passe à travers un perçage (14) dans la première surface de contact (6) et dans la deuxième surface de contact (10).

4. Système de fixation selon l'une quelconque des revendications précédentes, **caractérisé en ce que** l'élément de serrage présente une plaque à pas de vis (20) qui peut être positionnée sur l'un des côtés d'un composant (2, 4) qui se trouve à l'opposé de la surface de contact (6, 10) correspondante.

5. Système de fixation selon la revendication 4, **caractérisé en ce que** l'élément de serrage, en particulier la plaque à pas de vis (20), présente au moins une butée (28) qui, à l'état fixé, est en contact avec au moins une contre-butée (30) sur l'un des composants (2, 4).

6. Système de fixation selon l'une quelconque des revendications 3 à 5, **caractérisé en ce que** l'élément de serrage passe à travers l'élément de transmission des forces (18) lorsque les deux composants (2, 4) se trouvent à l'état fixé.

7. Système de fixation selon l'une quelconque des revendications précédentes, **caractérisé en ce que** l'élément de transmission des forces (18) est constitué d'un matériau élastique, par exemple de caoutchouc.

8. Système de fixation selon l'une quelconque des revendications précédentes, **caractérisé en ce qu'**une rainure (16) destinée à recevoir l'élément de transmission des forces (18) est ménagée dans la première surface de contact (6) et/ou la deuxième surface de contact (10).

9. Système de fixation selon l'une quelconque des revendications précédentes, **caractérisé en ce que** le dispositif orthopédique technique est une articulation.
